(19) Europäisches Patentamt
European Patent Office
Office européen des brevets

(11) Veröffentlichungsnummer: **0 282 708**
**B1**

(12) # EUROPÄISCHE PATENTSCHRIFT

(45) Veröffentlichungstag der Patentschrift: **05.09.90**

(51) Int. Cl.[5]: **C07D 301/12**, C07D 303/04

(21) Anmeldenummer: **88101284.3**

(22) Anmeldetag: **29.01.88**

(54) **Verfahren zur katalytischen Epoxidation von Olefinen mit Wasserstoffperoxid.**

(30) Priorität: **13.02.87 DE 3704532**

(43) Veröffentlichungstag der Anmeldung:
**21.09.88 Patentblatt 88/38**

(45) Bekanntmachung des Hinweises auf die Patenterteilung:
**05.09.90 Patentblatt 90/36**

(84) Benannte Vertragsstaaten:
**AT BE CH DE FR GB IT LI NL SE**

(56) Entgegenhaltungen:

**US-A- 3 156 709**

**J. AM. CHEM. SOC., Band 103, Nr. 12, 1981, Seiten 3601-3603, American Chemical Society; H.J. LEDON et al.: "Selective epoxidation of olefins by molybdenum porphyrin catalyzed peroxy-bond heterolysis"**

**F. Varescon, These, L'université Claude Bernard- Lyon I, 1982**

(73) Patentinhaber: **Degussa Aktiengesellschaft, Weissfrauenstrasse 9, D-6000 Frankfurt am Main 1(DE)**

(72) Erfinder: **Schmidt, Manfred, Dr., Unter-Haitzergasse 19, D-6460 Gelnhausen(DE)**
Erfinder: **Prescher, Günter, Dr., Liesingstrasse 2, D-6450 Hanau 9(DE)**

**EP 0 282 708 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur katalytischen Epoxidation von Olefinen mit Wasserstoffperoxid in Anwesenheit eines Übergangsmetallprophyrinkomplexes, wobei man das Olefin in Gegenwart eines Molybdänoxo-Komplexes mit 5,10,15,20-Tetraphenylporphyrin als Liganden in Form

- einer dimeren Dioxoverbindung der Formel µ-Oxo[Mo-oxo-(5,10,15,20-tetraphenylporphyrin)]$_2$,
- einer Bisperoxoverbindung der Formel trans-Diperoxomolybdän(VI)-5,10,15,20-tetraphenylporphyrin
- eines cis-Dioxo-5,10,15,20-tetraphenylporphyrinatomolybdän(VI)-Komplexes

in homogener Phase oder in einem Zweiphasensystem mit Wasserstoffperoxid umsetzt, wobei an dem Liganden 5,10,15,20-Tetraphenylporphyrin jeweils Wasserstoffatome der Phenylgruppen ein- oder mehrmals substituiert sind.

Olefinoxide (Oxirane) sind Verbindungen von beträchtlicher industrieller Bedeutung. Sie finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe US PS 3 412 136 sowie DE AS 1 139 477).

Zur Epoxidation von Olefinen sind schon verschiedene Verfahren bekannt. So lassen sich Oxirane nach der Chlorhydrinmethode durch Umsetzung von Olefinen mit Chlor oder Natriumhypochlorit im alkalischen Medium und nachfolgender Behandlung mit Basen herstellen. Ein grundsätzlicher Nachteil dieses Verfahrens besteht in der Bildung salzhaltiger umweltbelastender Abwässer und unerwünschter chlorierter Nebenprodukte (siehe Ullmann's Enzyklopädie der technischen Chemie, Band 10, Seite 565 (3. Auflage).

Ein weiterer Prozeß beruht auf der Umsetzung von Olefinen mit organischen Hydroperoxiden in Gegenwart eines Katalysators (siehe DE AS 1 468 012). Dieses zweite Syntheseprinzip hat den entscheidenden Nachteil, daß aufgrund der Stöchiometrie der Epoxidationsreaktion das üblicherweise teure organische Hydroperoxid (ROOH, wobei R z.B. einen niedermolekularen Rest, wie t-Butyl oder Cumyl, bedeuten kann), während der Reaktion gemäß

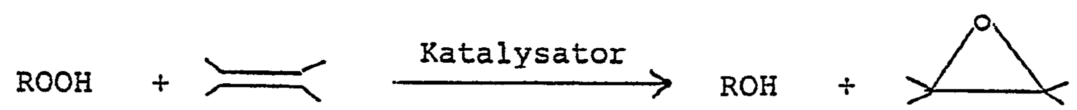


in große Mengen des entsprechenden Alkohols (ROH) umgewandelt wird. Falls der entsprechende Alkohol nicht verwertet werden kann, muß er vom Verfahrensprodukt abgetrennt und entsorgt oder über mehrere Verfahrensstufen in das entsprechende Hydroperoxid zurückverwandelt werden, wodurch das Epoxidationsverfahren auch in wirtschaftlicher Hinsicht aufwendig wird.

Ein weiteres Verfahren beruht auf der Verwendung von organischen Persäuren, die man durch Luftoxidation der entsprechenden Aldehyde oder aus Carbonsäuren mit Wasserstoffperoxid erhält (siehe BE PS 535 068). Der Einsatz dieser organischen Percarbonsaüren ist wegen deren Zersetzlichkeit stets mit einem Risiko behaftet und erfordert deshalb aufwendige Vorsichtsmaßnahmen in bezug auf Verfahrensführung und Apparateaufbau. Zusätzlich entstehen bei Epoxidierungen mit organischen Persäuren immer große Mengen der entsprechenden Carbonsäuren, die in stöchiometrischer Menge oder überstöchiometrischer Menge abgetrennt und entsorgt oder zurückgeführt werden müssen.

Die geschilderten Nachteile lassen sich durch Verwendung von Wasserstoffperoxid als Epoxidationsmittel beheben, da hierbei nach der Theorie neben dem Epoxidationsprodukt nur Wasser anfallen sollte. Da die Reaktivität des Wasserstoffperoxids gegenüber Olefinen schwach ist, werden Epoxidationen mit diesem Reagens unter Verwendung von Katalysatoren durchgeführt. Nur für wenige Olefine sind Katalysatoren wie Molybdän- und Wolframverbindungen geeignet. In diesem Zusammenhang wird beispielsweise hingewiesen auf GB PS 837 464, bei welchem die in J.A.C.S., Band 59, Seiten 2342 bis 2344 (1937) beschriebenen verschiedenen Metallkatalysatoren verwendet werden, auf US PS 2 786 854, wonach Wolframsäure eingesetzt wird, auf US PS 2 833 787, wonach saure Salze von Metallen aus der Gruppe VI des Periodensystems der Elemente, z.B. von Wolfram oder Molybdän, angewandt werden, auf BE PS 860 776, wonach Wolfram- und Molybdänhaltige Verbindungen verwendet werden, auf US PS 3 993 673, wonach Arsen-haltige Katalysatoren verwendet werden, auf US PS 3 953 362, wonach ein Molybdän-haltiger Katalysator angewandt wird, auf US PS 4 026 908, wonach Quecksilberderivate plus eine Molybdän, Wolfram, Vanadin- oder Titan-Verbindung angewandt wird, auf US PS 3 806 467, wonach organische und anorganische Zinnverbindungen plus organische oder anorganische Verbindungen, die Molybdän, Wolfram, Vanadin, Selen oder Bor enthalten, eingesetzt werden, auf Bull. Chem. Soc. Jap. 42, Seiten 1604 (1969), wonach Selendioxid angewandt wird und auf US PS 3 778 451, wonach Molybdän, Wolfram, Vanadin-, Niob-, Tantal-, Uran- und Rheniumverbindungen eingesetzt werden.

Diese Stoffe sind zwar katalytisch aktiv, doch haben ans verschiedenen Gründen die damit betriebenen Verfahren keinen Eingang in die Technik gefunden. In Verbindung mit Wasserstoffperoxidlösungen

wird durch sie entweder das Wasserstoffperoxid rasch zersetzt oder die Expoxidationsgeschwindigkeit unwirtschaftlich verlängert. Verfahren mit diesen Katalysatoren sind auch insoweit problematisch, als neben dem gewünschten Epoxidationsprodukt häufig größere Mengen an Nebenprodukten, wie Diole und Ketone gebildet werden, deren Abtrennung erhebliche Schwierigkeiten bereiten kann.

Es sind auch schon Versuche unternommen worden, Verfahren zur katalytischen Epoxidation von Olefinen mit anderen Epoxidationsmitteln unter Verwendung von Metallporphyrinkomplexen als Katalysatoren durchzuführen. Als Epoxidationsmittel wurden hierbei Verbindungen wie Jodosobenzol PhJO (Groves, J.T.; Wemo, T.E.; Myers, R.S, J. Am. Chem. Soc., 101, 1032 (1979), Alkalimetallhypochlorit, wie NaOCl oder LiOCl (Guilmet, E.; Meunier, B.; Tetrahedron Lett. 1980, 4449) sowie organische Hydroperoxide, wie t-Butylhydroperoxid oder Cumolhydroperoxid (Ledon, H.J.; Durbut, P.; Varescon, F., J. Am. Chem. Soc. 103, 3601 (1981) eingesetzt. Als zur Umsetzung mit diesen Epoxidationsmitteln geeignete Metallkatalysatoren sind z.B. das Chloro-Eisen(III)-tetraphenylporphyrin (ClFe(III)(TPP)), das Chloro-Mangan(III)-tetraphenylporphyrin (ClMn(III)(TPP)) oder das Chloro-Chrom(III)-tetraphenylporphyrin (ClCr(III)(TPP)) vorgeschlagen worden. Mangan(III)-tetraphenylporphyrin wurde auch bereits mit Wasserstoffperoxid als Oxidationsmittel eingesetzt (Renaud, J.-P.; Battioni, P.; Bartoli, J.F.; Mansuy, D., J. Chem. Soc., Chem. Commun.,1985, 888). Allerdings wirken diese stark zersetzend auf $H_2O_2$, so daß die bezüglich Wasserstoffperoxid erreichbaren Selektivitäten nur sehr gering sind.

Auch Oxo-Metallporphyrinkomplexe, wie Oxo-chloro(5,10,15,20-tetraphenylporphyrin)-Molybdän(V) (O=MO(V)(TPP)Cl) sind in Verbindung mit organischen Hydroperoxiden schon vorgeschlagen worden. Ein Versuch, anstelle eines organischen Hydroperoxids Wasserstoffperoxid mit einem Katalysator der Zusammensetzung Oxo(5,10,15,20-tetraphenylporphyrin)-Molybdän(V) -methoxid zur Epoxidierung des Olefins Cyclohexen zu verwenden, schlug jedoch fehl: Es konnte keine Epoxidation beobachtet werden (F.Varescon, These, L'Université Claude Bernard-Lyon I, 1982).

In der älteren, unter 54 (3) EPÜ fallenden Anmeldung gemäß EP-A 222 167 ist bereits ein Verfahren angegeben zur katalytischen Epoxidation von Olefinen und Wasserstoffperoxid in Anwesenheit eines Übergangsmetallporphyrinkomplexes, der gegebenenfalls in 5,10,15,20-Stellung des Porphyrinringes arylsubstituiert ist und bei dem ein erforderlicher Ladungsausgleich durch ein Anion erfolgt.

Das Verfahren besteht darin, daß man das Olefin in Gegenwart eines Molybdänkomplexes bzw. Molybdän-oxo-Komplexes mit Oktaethylporphyrin oder mit Liganden der Formel

- 5,10,15,20-Tetraphenylporphyrin,
- 5, 10, 15, 20-Tetra(4)-pyridyl)-porphyrin

bei denen gegebenenfalls jeweils Wasserstoffstome an den Phenyl- bzw. Pyridylgruppen ein- oder mehrmals substituiert sind durch Halogen, Hydroxy, Carboxy, Cyano, Rhodano, Nitro, $C_1$–$C_6$-Alkyl, Trihalogenmethyl, $C_1$--$C_6$-Alkoxy, $C_1$-$C_6$-Alkansulfonyloxy, Aminocarbonyl, einen oder zwei $C_1$–$C_6$-Alkylreste enthaltendes Aminocarbonyl, $C_1$–$C_6$-Alkylcarbonyl, Amino, Di-$C_1$–$C_6$-Alkylamino, ($C_1$–$C_6$-Alkyl)$_3$N, $C_1$–$C_6$-Alkanoylamino, $C_1$–$C_6$-Alkyl-$C_1$–$C_6$-alkanoylamino, $C_1$–$C_6$-Alkansulfonylamino, $C_1$–$C_6$-Alkyl-$C_1$–$C_6$-alkansulfonylamino, Aminosulfonyl, einen oder zwei $C_1$–$C_6$-Alkylreste enthaltendes Aminosulfdonyl, $C_1$–$C_6$-Alkoxysulfonyl(-$SO_2O$-$C_1$–$C_6$-Alkyl), Sulfo oder $C_1$–$C_6$-Alkansulfonyl und zwei dieser Reste auch die Methylendioxygruppe sein können, wobei der Komplex jeweils am Zentralatom ein Anion aus der Reihe $F^-$, $C1^-$, $Br^-$, $J^-$, $CH_3O^-$, $C_2H_5O^-$, $CH_3H_7O^-$, t-$C_4H_9O^-$, $HO^-$, $AcO^-$, $SCN^-$, $C_6H_5O^-$, pyridyl trägt, oder in Gegenwart

- einer dimeren Dioxoverbindung der Formel µ-Oxo[Mo-oxo-(5,10,15,20-tetraphenylporphyrin]$_2$.
- einer Bisperoxoverbindung der Formel transDiperoxomolybdän(VI)porphyrin oder
- eines cis-Dioxoporphyrintomolybdän(VI)-Komplexes

in homogener Phase oder in einem Zweiphasensystem mit Wasserstoffperoxid umsetzt.

Hierbei war noch nicht erkannt, daß bei Einsatz des bereits vorgeschlagenen dimeren Molybdän-oxo-Komplexes mit 5,10,15,20-Tetraphenylporphyrin als Liganden eine Substitution von Wasserstoffatomen in den Phenylringen des Liganden technische Vorteile erbringt.

Es wurde also gefunden, daß die genannte und in der vorstehend erwähnten europäischen Patentanmeldung offenbarte katalytische Epoxidation mit hoher Selektivität und verbesserter Anpaßbarkeit an das jeweilige Olefin gelingt, wenn an dem Liganden 5,10,15,20-Tetraphenylporphyrin jeweils Wasserstoffatome der Phenylgruppen ein- oder mehrmals substituiert sind durch Halogen, Hydroxy, Carboxy, Cyano, Rhodano, Nitro, $C_1$–$C_6$-Alkyl, Trihalogenmethyl, $C_1$–$C_6$-Alkoxy, $C_1$–$C_6$-Alkansulfonyloxy, Aminocarbonyl, einen oder zwei $C_1$–$C_6$-Alkylreste enthaltendes Aminocarbonyl, $C_1$–$C_6$-Alkylcarbonyl, Amino, Di-$C_1$–$C_6$-Alkylamino, $C_1$–$C_6$-Alanoylamino, $C_1$–$C_6$-Alkyl-$C_1$–$C_6$-Alkanoylamino, $C_1$–$C_6$-Alkansulfonylamino, $C_1$–$C_6$-Alkyl-$C_1$–$C_6$-alkansulfonylamino, Aminosulfonyl, einen oder zwei $C_1$–$C_6$-Alkylreste enthaltendes Aminosulfonyl, $C_1$–$C_6$-Alkoxysulfonyl(-$SO_2$-O-$C_1$–$C_6$-Alkyl), Sulfo oder $C_1$–$C_6$-Alkansulfonyl und zwei dieser Reste auch die Methylendioxygruppe sein können.

Durch sterische und elektronische Effekte der Substituenten am Phenylrest des 5, 10, 15, 20-Tetraphenylporphyrins kann man, angepaßt an das jeweilige Olefin, die katalytischen Eigenschaften steuern.

Nach dem erfindungsgemäßen Verfahren können Olefine entsprechend der allgemeinen Formel

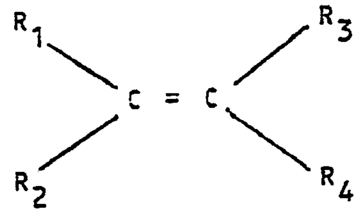

epoxidiert werden, wobei $R_1$ bis $R_4$ identisch oder verschieden sein können und sowohl Wasserstoff oder einen linearen oder verzweigten Alkylrest mit 1 bis 30 Kohlenstoffatomen oder einen Cycloalkylrest mit 3 bis 12 Kohlenstoffatomen oder Aromaten bzw. Heteroaromaten mit 3 bis 30 C-Atomen, die als Heteroatome z. B. ein- oder mehrere O-, N- oder S-Atome enthalten können, bedeuten. $R_1$ und $R_2$ oder $R_3$ und $R_4$ können auch durch funktionelle Gruppen substituiert sein, welche im Reaktionsmilieu stabil sind, wie z. B. durch Hydroxy-, Chloro-, Fluoro-, Bromo-, Jodo-, Nitro-, Methoxy-, Alkoxy-, Amino-, Carbonyl-, Ester-, Amido-, Nitrilo-gruppen. Sie können auch ungesättigt sein, d. h. daß Polyolefine, wie z. B. Diene, Triene und andere Verbindungen mit Doppelbindungen ebenfalls in der vorliegenden Erfindung verwendet werden können, seien sie konjugiert oder nicht.

Unter dieser Voraussetzung kommen unter den Olefinen, welche nach dem vorliegenden Verfahren epoxidiert werden können, beispielsweise die folgenden in Betracht:

Ethylen, Propylen, die Butene, Butadien, die Pentene, Isopren, Hexen(1), Hexen(3), Hepten(1), Okten(1), Diisobutylen, Nonen(1), Tetradecen(1), Pentamyrcen, Camphen, Undecen(1), Dodecen(1), Tridecen(1), Tetradecen(1), Pentadecen(1), Hexadecen(1), Heptadecen(1), Oktadecen(1), Nonadecen(1), Eikosen(1), die Trimeren und Tetrameren des Propylens, die Polybutadiene, die Polyisoprene, Styrol, α-Methylstyrol, Divinylbenzol, Inden, Stilben, Cyclopenten, Cyclohexen, Cyclohepten, Cyclooctadien, Cyclododecen, Cyclododekatrien, Dicyclopentadien, Methylencyclopropan, Methylencyclopentan, Methylencyclohexan, Vinylcyclohexen, Methallylketon, Allylchlorid, Allylbromid, Arylsäure, Methacrylsäure, Crotonsäure, Vinylessigsäure, Crotylchlorid, Methallylchlorid, die Dichlorbutene, Allylalkohol, Allylkarbonat, Allylacetat, die Alkyle der Acrylate und Methacrylate, Diallylmaleat, Diallylphthalat, die ungesättigten Öle wie Sojaöl, die ungesättigten Fettsäuren wie Ölsäuren, Linolensäure, Balidinsäure, Erucasäure, Oleostearinsäure, Myristinsäure, Palmitinölsäure, Ricinolsäure und deren Ester.

Ein Vorteil der Erfindung besteht darin, daß sich hierbei das als Reaktant benötigte Wasserstoffperoxid in allen handelsüblichen Formen verwenden läßt, nämlich in Form wäßriger Wasserstoffperoxidlösungen mit einem Wasserstoffperoxidgehalt von 30 bis 90 Gew.% oder als reines Wasserstoffperoxid, stärker verdünntes Wasserstoffperoxid, in organischen Lösungsmitteln gelöstes wasserfreies Wasserstoffperoxid oder in Form von Verbindungen, die unter den Reaktionsbedingungen Wasserstoffperoxid freisetzen (Metallperoxide), wie Magnesium- oder Zinkperoxid sowie Wasserstoffperoxid-Anlagerungsverbindungen (Peroxohydrate), z. B. von Natriumcarbonat, Natriumpyrophosphat und Harnstoff).

Besonders vorteilhaft ist, wenn man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

Als organische Lösungsmittel können dafür z. B. Alkyl- oder Cycloalkylester von gesättigten aliphatischen Carbonsäuren mit einer Kohlenstoffzahl von 4 - 8 (siehe z. B. DE-PS 3 225 307, Seite 5), Methylenchlorid, Dioxan, tert.-Butanol, Tetrahydrofuran, Benzol, Äthanol, Chloroform und Methanol verwendet werden.

Als Lösungsmittelgemische kommen z. B. Kombinationen aus einem oder mehreren der obengenannten Carbonsäureester mit Wasser, Methylenchlorid, Dioxan, tert.-Butanol, Tetrahydrofuran, Benzol, Äthanol, Chloroform und/oder Methanol in Frage.

Für die Lösung von wasserfreiem Wasserstoffperoxid hat sich ein Einsatz von Alkyl- oder Cycloalkylestern von gesättigten aliphatischen Carbonsäuren mit einer Kohlenstoffzahl von 4–8 besonders bewährt.

Die anzuwendenden Mengen an Katalysator, die im erfindungsgemäßen Verfahren eingesetzt werden, können in einem weiten Bereich liegen. Die im Einzelfall anzuwendende Katalysatorkonzentration kann, entsprechend dem Typ der gewählten, gemäß Erfindung vorgesehenen Molybdänporphyrinverbindung sowie entsprechend der Reaktivität des jeweils umzusetzenden Olefins gewählt werden. Sie liegt in einem Konzentrationsbereich, der bei 1/5000 mol pro mol Wasserstoffperoxid beginnt und im allgemeinen 1/2000 bis 1/2 mol, vorzugsweise 1/1000 bis 1/5 mol pro mol Wasserstoffperoxid beträgt.

Nach einer anderen vorteilhaften Ausführungsform der Erfindung können die ohnehin hohen Selektivitätsraten durch Zusatz von geringen Mengen, vorzugsweise von 0,1 bis 10 mol eines heterocyclischen Amins, bezogen auf 1 mol des Katalysators, insbesondere von äquimolaren Mengen, einer Verbindung aus der Familie des Pyridins, wie 2,6-Dimethylpyridin, 2,6-Ditertiärbutylpyridin, 3,5-Dimethylpyridin sowie die drei Picoline, die 4-Halogenopyridine sowie die Salze des 2,2-Bipyridyls, oder des Imidazols, z. B. das Imidazol selbst, verbessert werden. Die Konzentration des Olefins im Reaktionssystem ist nicht kritisch, das molare Verhältnis zwischen Olefin und Wasserstoffperoxid kann 1 : 30 bis 30 : 1 betragen.

Die Reaktionstemperaturen können in einem breiten Bereich liegen. Sie hängen ab von der jeweiligen Aktivität des verwendeten Katalysators, der Reaktivität des verwendeten Olefins, der Neigung des gewünschten Oxirans zur Ringöffnung und der Art des Lösungsmittels. Sie liegen im allgemeinen bei 0 bis 150, vorzugsweise 20 bis 120, insbesondere 40 bis 80° C. Die Reaktionszeiten sind kurz und liegen normalerweise bei 1 bis 24 Stunden. Die Reaktionen können unter Atmosphärendruck oder bei höheren Drucken durchgeführt werden, solange das Reaktionssystem in flüssiger Phase gehalten werden kann.

Vorzugsweise wird in einem Druckbereich zwischen 1 und 50 bar gearbeitet.

Die erfindungsgemäß zur Durchführung des Verfahrens vorgesehenen Katalysatoren sind nach den bekannten Literaturmethoden in großer Reinheit zugänglich (H. Ledon et al., J.Chem. Soc., Chem. Commun. 1979, 702; J.W. Buchler et al., Inorg. Nucl. Chem. Lett., 1972, 8, 1073; R. Weiss et al., Inorg. Chim. Acta, 1976, 19, L 57; F Varescon, These, L'Université Claude Bernhard-Lyon, 1982).

Die verschiedenen Porphyrinliganden werden, sofern sie nicht käuflich sind, nach Adler et al., J. Org. Chem. 32, 476 (1967) bzw. Adler et al., J. Heterocycl. Chem. 5, 669 (1968) dargestellt und sofern erforderlich, von Chlorin (Porphyrin mit 1 teilhydrierten Pyrrolglied) befreit (K.M. Smith et al., Tetrahedron Lett. 30, 2887, (1973).

Die mit der Erfindung erzielbaren Vorteile sind:

- Hohe Selektivität (kaum Nebenprodukt)
- Niedrige Katalysatorkonzentration
- Hohe chemische Stabilität des Katalysators, insbesondere gegenüber dem Epoxidationsmittel
- Keinerlei $H_2O_2$-Zersetzung
- Aus dem Epoxidationsmittel entsteht nur Wasser
- Leichte Abtrennbarkeit und Wiederverwendbarkeit des Katalysators
- Einfacher Verfahrensgang

Die Erfindung wird im folgenden anhand von Ausführungsbeispielen näher erläutert.

Vorbemerkung zu den einzelnen Beispielen:

Die in den Ausführungsbeispielen verwendeten, nach der Literatur hergestellten Katalysatoren werden zur Epoxidation unterschiedlicher olefinischer Ausgangsstoffe mit Wasserstoffperoxid gemäß der Erfindung wie folgt eingesetzt:

Man stellt eine Lösung aus Olefin, Katalysator und Lösungsmittel her, erwärmt sie auf eine Temperatur im Bereich von 20–100° C und versetzt mit Wasserstoffperoxid (30 bis 90 Gew.%) - Version I - oder man stellt eine Lösung aus Olefin, Wasserstoffperoxid (30 bis 90 Gew.%) und Lösungsmittel her und versetzt diese dann mit Katalysator; dann wird unter Rühren und Erwärmen umgesetzt - Version II.

In einer abgewandelten Ausführungsform, die sich auf die Versionen I und II gleichermaßen anwenden läßt, wird den vorgelegten Komponenten vor Zugabe der restlichen Komponente noch eine kleine Menge eines heterocyclischen Amins, z. B. aus der Familie des Pyridins oder Imidazols zugesetzt. Bei den eingesetzten Olefinen kann unter atmosphärischem Druck gearbeitet werden. Im Verlaufe der Umsetzung werden Proben entnommen und auf ihren Gehalt an Epoxid bzw. $H_2O_2$ analysiert. Die Menge an gebildeten Epoxiden wird entweder durch Gaschromatographie oder Titration, diejenige an Wasserstoffperoxid durch übliche Titration mit Cer(IV)-sulfat ermittelt. Die bei den Versuchen erhaltenen Ergebnisse gehen aus der folgenden Tabelle hervor, wobei die Selektivität wie folgt definiert ist:

$$\text{Selektivität (\%)} = \frac{\text{Mol gebildetes Epoxid}}{\text{Mol umgesetztes } H_2O_2} \times 100$$

| Beispiel | Olefin (mmol) | $H_2O_2$ (mmol) | Katalysator | (mmol) | Reaktionsbedin. Solvent (ml) | RT (°C) | RZ (h) | Umsatz $H_2O_2$ (%) | Selekt. (%) |
|---|---|---|---|---|---|---|---|---|---|
| 1 | 1,5-COD[1] (72) | 85% in $H_2O$ (24) | μ-O [OMo(TpTP)[2]]$_2$ | 24 x $10^{-2}$ | nPAC[3] (30) | 60 | 12 | 88,7 | 80,9 |
| 2 | Cyclohexen (72 | 85% in $H_2O$ (24) | $Mo(O_2)_2$(ToClPP)[4] | 24 x $10^{-2}$ | nPAC/γ-Pic[5] 30 0,1 | 60 | 24 | 74,0 | 89,4 |
| 3 | 2-Methyl-2-buten (72) | 25,9% in nPAC (24) | cis-Dioxo-Mo (VI)(ToNO$_2$PP)[6] | 24 x $10^{-2}$ | PME[7] 30 | 60 | 24 | 69,4 | 51,6 |

1) 1,5-Cyclooctadien
2) 5,10,15,20-Tetra(p-tolyl)-porphyrin
3) Essigsäurepropylester
4) 5,10,15,20-Tetra(o-chlorphenyl)porphyrin
5) γ-Picolin
6) 5,10,15,20-Tetra(o-nitrophenyl)porphyrin
7) Pivalinsäuremethylester

**Patentansprüche**

1. Verfahren zur katalytischen Epoxidation von Olefinen mit Wasserstoffperoxid in Anwesenheit eines Übergangsmetallporphyrinkomplexes, wobei man das Olefin in Gegenwart eines Molybdän-oxo-Komplexes mit 5,10,15,20-Tetraphenylporphyrin als Liganden in Form
- einer dimeren Dioxoverbindung der Formel μ-Oxo[Mo-oxo-(5,10,15,20-tetraphenylporphyrin)]$_2$,
- einer Bisperoxoverbindung der Formel trans-Diperoxomolybdän(VI)-5,10,15,20-tetraphenylporphyrin oder
- eines cis-Dioxo-5,10,15,20-tetraphenylporphyrinatomolybdän(VI)-Komplexes

in homogener Phase oder in einem Zweiphasensystem mit Wasserstoffperoxid umsetzt, dadurchgekennzeichnet, daß an dem Liganden 5,10,15,20-Tetraphenylporphyrin jeweils Wasserstoffatome der Phenylgruppen einoder mehrmals substituiert sind durch Halogen, Hydroxy, Carboxy, Cyano, Rhodano, Nitro, $C_1$–$C_6$-Alkyl, Trihalogenmethyl, $C_1$–$C_6$-Alkoxy, $C_1$–$C_6$-Alkansulfonyloxy, Aminocarbonyl, einen oder zwei $C_1$–$C_6$-Alkylreste enthaltendes Aminocarbonyl, $C_1$–$C_6$-Alkylcarbonyl, Amino, Di-$C_1$–$C_6$-Alkylamino, $C_1$–$C_6$-Alkanolaymino, $C_1$–$C_6$-Alkyl-$C_1$–$C_6$-alkanoylamino, $C_1$–$C_6$-Alkansulfonylamino, $C_1$–$C_6$-Alkyl-$C_1$–$C_6$-alkansulfonylamino, Aminosulfonyl, einen oder zwei $C_1$–$C_6$-Alkylreste enthaltendes Aminosulfonyl, $C_1$–$C_6$-Alkoxysulfonyl(–$SO_2$-O-$C_1$-$C_6$-Alkyl), Sulfo oder $C_1$–$C_6$-Alkansulfonyl und zwei dieser Reste auch die Methylendioxygruppe sein können.

2. Verfahren nach Anspruch 1, dadurch gekennzeichnet, daß man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

3. Verfahren nach Anspruch 1 oder 2, dadurch gekennzeichnet, daß man bei Einsatz von wasserfreiem Wasserstoffperoxid als organische Lösungsmittel Alkyl- oder Cycloalkylester von gesättigten aliphatischen Carbonsäuren mit einer Kohlenstoffzahl von 4 - 8 einsetzt.

4. Verfahren nach den Ansprüchen 1 bis 3, dadurch gekennzeichnet, daß die Umsetzung in Gegenwart von 0,1 bis 10 mol, vorzugsweise 0,5 - 5 mol, bezogen auf 1 mol des Katalysators, einer Verbindung aus der Familie des Pyridins oder Imidazols erfolgt.

5.Verfahren nach Anspruch 4, dadurch gekennzeichnet, daß γ-Picolin oder Imidazol eingesetzt wird.

**Claims**

1. A process for for the catalytic epoxidation of olefins with hydrogen peroxide in the presence of a transition metal porphyrin complex, in which the olefin is reacted with hydrogen peroxide in the presence of a molybdenum-oxo complex with 5,10,15,20-tetraphenyl porphyrin as ligand in the form of
- a dimeric dioxo compound corresponding to the formula μ-oxo[Mo-oxo-(5,10,15,20-tetraphenylporphyrin)]$_2$,
- a bisperoxo compound corresponding to the formula trans-diperoxomolybdenum(VI)-5,10,15,20-tetraphenylporphyrin or
- a cis-dioxo-5,10,15, 20-tetraphenylporphyrinatomolybdenum(VI) complex

in homogeneous phase or in a two-phase system, characterized in that, at the ligand 5,10,15,20-tetraphenyl porphyrin, hydrogen atoms of the phenyl groups are substituted one or more times by halogen, hydroxy, carboxy, cyano, thiocyano, nitro, $C_{1-6}$ alkyl, trihalomethyl, $C_{1-6}$ alkoxy, $C_{1-6}$ alkanesulfonyloxy, aminocarbonyl, aminocarbonyl containing one or two $C_{1-6}$ alkyl groups, $C_{1-6}$-alkyl carbonyl, amino, di-$C_{1-6}$-alkylamino, $C_{1-6}$-alkanoylamino, $C_{1-6}$-alkyl-$C_{1-6}$-alkanoylamino, $C_{1-6}$-alkanesulfonylamino, $C_{1-6}$-alkyl-$C_{1-6}$-alkanesulfonylamino, aminosulfonyl, aminosulfonyl containing one or two $C_{1-6}$-alkyl groups, $C_{1-6}$-alkoxysulfonyl (–$SO_2O$-$C_{1-6}$-alkyl), sulfo or $C_{1-6}$ alkanesulfonyl and two of these radicals may also be the methylenedioxy group.

2. A process as claimed in claim 1, characterized in that the reaction medium used is an organic solvent or solvent mixture which enables hydrogen peroxide used in the form of an aqueous solution to pass into the organic phase.

3. A process as claimed in claim 1 or 2, characterized in that, where anhydrous hydrogen peroxide is used, alkyl or cycloalkyl esters of saturated aliphatic carboxylic acids containing 4 to 8 carbon atoms are used as the organic solvent.

4. A process as claimed in claims 1 to 3, characterized in that the reaction is carried out in the presence of 0.1 to 10 mol and preferably 0.5 to 5 mol, based on 1 mol of the catalyst, of a compound from the pyridine or imidazole family.

5. A process as claimed in claim 4, characterized in that γ-picoline or imidazole is used.

**Revendications**

1. Procédé pour l'époxydation catalytique d'oléfines avec du peroxyde d'hydrogène en présence d'un complexe de porphyrine avec un métal de transition, dans lequel on fait réagir l'oléfine avec du peroxyde d'hydrogène, en phase homogène ou dans un système à deux phases, en présence d'un oxo-complexe de

molybdène avec de la 5, 10, 15, 20-tétraphénylporphyrine en tant que ligand, sous forme :

- d'un composé dioxo dimère de formule µ-oxo[Mo-oxo-(5, 10, 15, 20-tétraphénylporphyrine)]$_2$,
- d'un composé bis-peroxo de formule trans-diperoxomolybdène(VI)-5, 10, 15, 20-tétraphénylporphyrine ou
- d'un complexe cis-dioxo-5, 10, 15, 20-tétraphénylporphyrinatomolybdène(VI),

caractérisé en ce qu'un ou plusieurs atomes d'hydrogène des groupes phényle sur le ligand 5, 10, 15, 20-tétraphénylporphyrine sont remplacés chacun par un halogène ou par un groupe hydroxy, carboxy, cyano, thiocyanato, nitro, alkyle en $C_1$–$C_6$, trihalogénométhyle, alcoxy en $C_1$–$C_6$, alcane ($C_1$–$C_6$)-sulfonyloxy, aminocarbonyle, un groupe aminocarbonyle contenant un ou deux radicaux alkyle en $C_1$–$C_6$, un groupe alkyl($C_1$–$C_6$)-carbonyle, amino, dialkyl($C_1$–$C_6$)-amino, alconyl-($C_1$–$C_6$)-amino, alkyl($C_1$–$C_6$)-alcanoyl($C_1$–$C_6$)-amino, alcane($C_1$-$C_6$)-sulfonylamino, alkyl($C_1$–$C_6$)-alcane($C_1$–$C_6$)-sulfonylamino, aminosulfonyle, un groupe aminosulfonyle contenant un ou deux radicaux alkyle en $C_1$–$C_6$, un groupe alcoxy($C_1$–$C_6$)-sulfonyl(–$SO_2$-O-alkyle en $C_1$–$C_6$), sulfo ou alcane($C_1$–$C_6$)-sulfonyle, et deux de ces radicaux peuvent être aussi le groupe méthylènedioxy.

2. Procédé selon la revendication 1, caractérisé en ce que l'on utilise en tant que milieu réactionnel un solvant organique ou un mélange de solvants qui permet un passage dans la phase organique du peroxyde d'hydrogène introduit sous forme de solution aqueuse.

3. Procédé selon la revendication 1 ou 2, caractérisé en ce que, lorsqu'on utilise du peroxyde d'hydrogène anhydre, on utilise en tant que solvants organiques des esters alkyliques ou cycloalkyliques d'acides carboxyliques saturés ayant de 4 à 8 atomes de carbone.

4. Procédé selon l'une quelconque des revendications 1 à 3, caractérisé en ce que l'on effectue la réaction en présence de 0,1 à 10 moles, de préférence de 0,5 à 5 moles, par rapport à 1 mole du catalyseur, d'un composé de la famille de la pyridine ou de l'imidazole.

5. Procédé selon la revendication 4, caractérisé en ce que l'on utilise la γ-picoline ou l'imidazole.